# EUROPÄISCHE PATENTSCHRIFT

(11) **EP 0 699 442 B1**
(45) Veröffentlichungstag und Bekanntmachung des Hinweises auf die Patenterteilung: **16.09.1998**
(21) Anmeldenummer: 95903058.6
(22) Anmeldetag: 23.11.1994
(51) Int. Cl.: A61K 35/70

(54) **DIE VERWENDUNG EINES STAMMES DES PILZES FUSARIUM ZUR HERSTELLUNG VON SUBSTANZEN UND EINE DARAUF BASIERENDE ZUBEREITUNG MIT ADAPTIVEN UND IMMUNMODULIERENDEN EIGENSCHAFTEN**
USE OF A STRAIN OF THE FUNGUS FUSARIUM AS A PRODUCER OF SUBSTANCES AND A PREPARATION BASED ON SAID SUBSTANCES WITH ADAPTOGENIC AND IMMUNOMODULATING PROPERTIES
UTILISATION D'UNE SOUCHE DU CHAMPIGNON FUSARIUM POUR LA PRODUCTION DE SUBSTANCES, ET UNE PREPARATION BASEE SUR LESDITES SUBSTANCES AYANT DES PROPRIETES ADAPTATIVE ET IMMUNO-MODULATRICE

(30) Priorität: 21.12.1993 RU 93057876
(43) Veröffentlichungstag der Anmeldung: 06.03.1996
(73) Patentinhaber: Krestyanskoe Khozyaistvo "Agrofirma Dizha", Maikop, 352700 (RU)
(72) Erfinder: MOROZOVA, Galina Rostislavovna, RU-101000 Moscow (RU); MOROZOV, Alexandr Lvovich, RU-117418 Moscow (RU)
(74) Vertreter: Kindler, Matthias, Dr. Dipl.-Chem.
(86) Internationale Anmeldenummer: RU9400260
(87) Internationale Veröffentlichungsnummer: WO9517902

(56) Entgegenhaltungen:
- EP-A- 0 413 053
- EP-A- 0 610 499
- WO-A-87/02249
- FR-A- 2 384 501
- GB-A- 2 010 848
- DATABASE BIOSIS BIOSCIENCES INFORMATION SERVICE, PHILADELPHIA, PA, US ZUSAMMENFASSUNG 93:482332, BABAKHIN ET AL: "PHARMACOLOGICAL CONTROL OF ALLERGY AT THE STAGE OF IGE ANTIBODY PRODUCTION" XP002025730 & KHIM-FARM ZH, Bd. 27, Nr. 2, 1993, Seiten 4-7,
- DATABASE BIOSIS BIOSCIENCES INFORMATION SERVICE, PHILADELPHIA, PA, US ZUSAMMENFASSUNG 87:488513, POPRAVKO ET AL: "A COMPARITIVE STUDY OF THE FATTY ACID COMPOSITION OF ACYLGLYCERIDES OF THE MYCELIUM OF FUSARIUM-SAMBUCINUM-VAR-OSSICOLUM BERK. EX CURT." XP002025731 & PRIKL BIOKHIM MIKROBIOL , Bd. 23, Nr. 4, 1987, Seiten 478-485,
- MICROBIOLOGICAL SCIENCES, Bd. 3, Nr. 6, 1986, Seiten 168-171, XP000644746 CANNON: "INTERNATIONAL COMISSION ON THE TAXONOMY OF FUNGI (ICTF): NAME CHANGES IN FUNGI OF MICROBIOLOGICAL, INDUSTRIAL AND MEDICAL IMPORTANCE. PART 1."

## Beschreibung

Die vorliegende Erfindung betrifft die Mykologie und Medizin, insbesondere die Verwendung von bestimmten Pilzen zur Herstellung von physiologisch wirksamen Stoffen und Präparaten auf deren Basis für die Verwendung im Gesundheitswesen.

### Zugrundeliegender Stand der Technik

Den Fachleuten sind der Stamm des höheren Pilzes INONOTUS OBLIQUUS und das Präparat Bephungin, das auf deren Basis entwickelt worden war, bekannt.

Bephungin stellt einen Extrakt von Ablagerungen dar, die sich auf der Birke unter Einwirkung dieses Pilzes bilden. Das Präparat stellt eine halbdickflüssige fließende Masse dar, die 30% trockener Substanzen enthält, denen 1% Cobaltchlorids oder 1,5% Cobaltsulphat zugefügt sind. Es wird als symptomatisches Mittel verwendet, das das Befinden der Kranken mit chronischer Gastritis und Dyskinesien des Magen-Darm-Kanals mit Dominieren der Atonie verbessert (M.D. Maschkowsky, Arzneimittel, Verlag "Medizina", Moskau, 1984, B. 2, S. 162).

Aber die Ressourcen des Rohstoffes INONOTUS OBLIQUUS für die Herstellung von Bephungin sind überaus beschränkt, ebenso wie auch das Spektrum der physiologischen Einwirkung des Präparates.

Es ist genauso bekannt, daß die Pilze LENTINUS EDODES, GANODERMA LUCIDUM, PLEUROTIS OSTREATUS eine antisklerotische Wirkung besitzen und den Cholesterinspiegel im Organismus normalisieren (Li Chwa Rei, Wassiljew L. W., Orechow L. N., Tertow W. W., Tutjan W. A., Antisklerotische Eigenschaften von höheren Pilzen, Fragen der Ernährung Nr. 1, 1989, Seiten 16-20). Die erwähnten Pilze haben aber eine niedrige Wachstumsgeschwindigkeit : das Myzel wächst 5 bis 7 Tage, und der Fruchtkörper über 70 Tage, und besitzen ein schmales Spektrum der physiologischen Wirkung

Eine höhere Wachstumsgeschwindigkeit unter Ausnutzung von modernen Biotechnologien besitzen die Pilze der Art FUSARIUM.

Aus Database Biosis, Philadelphia, US, N5 93:482332 ist als Abstract von Khim-Farm ZH 27(2) 1993, 4-7 Babakhin et al, bekannt, daß Poliporus squamosus Stamm PS-64 bei der ersten Immunisierung die Bildung von IgE und IgG in Mäusen gegen Ovalbumin hemmt, und bei der zweiten Immunisierung IgE nicht beeinflußt und IgG steigert. Die vermutete Nutzbarkeit als Antiallergikum konnte nicht bestätigt werden.

Vorbekannt ist der Stamm des Pilzes FUSARIUM SAMBUCINUM FUCKEL VAR. OSSICOLUM (BERK. ET. CURT.) Bulai WSB-917, der ein Produzent vom Eiweiß mit der Nahrungs- oder Futterzweckbestimmung ist (Allunionskollektion von Industriemikroorganismen des Instituts WNIIgenetika, Kollektionsnummer WKPM F-169). Früher hatte dieser Stamm die Bezeichnung POLYPORUS SP. WSB-917 (Microbiol. Sciences, 1986, v.3, Nr. 6, P. 168-171).

Der Stamm F. SAMBUCINUM FUCKEL VAR. OSSICOLUM (BERK. ET CURT) BULAI WSB-917 wurde auf der Stufe des aktiven Wachsens im Prozeß der ununterbrochen ablaufenden Züchtung des Myzeliums vom Stamm F. SAMBUCINUM PS-64 (Allunionskollektion von Industriemikroorganismen des Instituts WNIIgenetika, Kollektionsnummer ZMPM-F-165) isoliert.

Der F. SAMBUCINUM WSB-917, F-169 weist die nachfolgenden kulturellen und physiologischen Merkmale auf:
auf Würzeagar bildet er weiße Filzkolonien, die sich bei der Alterung rosig färben, bildet aber keine Fruchtkörper, die Schnallen sind nicht festgestellt.
Er hat zwei Arten vom Sporentragen - Konidien und Chlamidosporen, sichelförmige Konidien mit 2 bis 5 Trennwänden.
Bei der Züchtung im flüssigen Nährboden bildet er das vielzellige fadenartige Myzelium mit den Trennwänden vom Askomyzetentyp mit den Woroninkörperchen.
In der Stufe des aktiven Wachsens beträgt die Dicke der Hyphen 2,5 bis 3,5, die Myzeliumzellen sind homogen, ohne Lipideinschlüsse oder andere Einschlüsse, die Enden der Hyphen (des Myzeliums) sind gleichmäßig abgerundet. Bei der Alterung erscheinen in den Zellen zahlreiche Fetteinschlüsse.

Der Stamm ist ein Aerobier, wächst im Temperaturbereich von 22 bis 28°C und im pH-Intervall von 4,5 bis 7,0, der optimale pH-Wert ist 5,8. Er verflüssigt die Gelatine nicht. Er assimiliert Glucose, Saccharose, Lactose, Maltose, Galaktose, Raffinose sowie Stärke, Ethanol, Glyzerin, Essigsäure, Mannit, Xylit.

Verhalten zur Stickstoffquelle: er assimiliert Ammoniumsalze, Nitrate, Harnstoff, Pepton.

Unter den Bedingungen der Tiefenzüchtung wächst er gut auf dem Milchserum (4-6 % Lactose), wobei die Ausbeute an ASB 20-25 g pro Liter des Nährbodens beträgt.

Die Produktivität beträgt beim semikontinuierlichem Prozeß 1,4 bis 1,6 kg/m³/Stunde.

Das Stammyzellium, das unter den Bedingungen der Tiefenzüchtung auf dem Nährboden erhalten wird, welcher in Gew.% enthält: Melasse - 4, Ammoniumnitrat - 0,3, saures Kaliumphosphat - 0,2, Rest Leitungswasser. Der pH-Wert des Mediums vor der Sterilisation beträgt 6,0-6,2, die Temperatur 26 bis 28°C. Nach einer Züchtung von 16 Stunden, unterwart man die Mischung einer Autolyse, und fügte das Autolysat in einer Menge von 0,25 bis 1,0 % dem Nährboden für das Wachsen der Hefe TORULOPSIS CANDIDA zu,(UdSSR-Urheberschein Nr. 1 034 401 vom 18.03.1982, C 12 Nr. 1/16), was zur Vergrößerung des Eiweißgehaltes in der Biomasse der Hefe sowie zur Erhöhung von deren Konzentration in dem Aufzuchtmedium von 31,1-32 g bis 34-44 ASW in 1 führte.

Die neue Verwendung des Stammes F. SAMBUCINUM WSB-917 ist die Herstllung von physiologisch wirksamen Stoffen mit prophylaktischer und therapeutischer Wirkung.

### Offenbarung der Erfindung

Der Erfindung betrifft die Verwendung des Stammes F. SAMBUCINUM WSB-917 zur Herstellung von einem Präparat, das ein weites Spektrum der physiologischen Wirkung im Organismus aufweist und keine Kontraindikation für die Verwendung hat.

Auf der Basis des obigen Stammes wurde ein Präparat erhalten, das ein weites Spektrum der heilend-vorbeugenden Wirkung durch die in diesem enthaltenden verschiedenen physiologisch wirksamen Stoffe: essentielle Aminosäuren, immunomodulierende Polysaccharide, essentielle Fettsäuren, biologische Amine, Ubichinone, Vitamine und Spurenelemente, die in den natürlichen Verhältnissen zueinander stehen, aufweist. Das Präparat übt eine normalisierende Wirkung auf die gestörten Stoffwechselprozesse im Organismus aus, die zu den kardiovaskulären Krankheiten, Fettleibigkeit, Diabetes, Immunodefizitzuständen und Vitaminmangelkrankheiten führen. Das Präparat ruft keine allergischen Reaktionen hervor, verbindet sich mit den traditionellen therapeutischen Mitteln, indem es ihre Wirkung auf den Organismus verstärkt und die ungünstigen Nebeneffekte, insbesondere bei der Durchführung der Strahlentherapie der onkologisch Kranken, vermindert.

Es sind untersucht: die chemische Zusammensetzung des Präparates und die möglichen Nebenwirkungen - Pathogenität, akute und chronische Toxizität, Teratogenität in fünf Generationen von Tieren, Carzinogenität, Cocartinogenität. Die Untersuchungen sind in Übereinstimmung mit den Forderungen der Weltorganisation des Gesundheitswesens (WHO) für die Prüfung der Stoffe des Mikrobiellen Ursprungs durchgeführt. Infolge der Durchführung dieser Serie von Experimenten ist das Präparat von den Behörden des Gesundheitswesens für die Anwendung genehmigt.

Das Präparat hat eine Warenbezeichnung "Milaif" erhalten.

Die chemische Zusammensetzung des Präparates MILAIF in Gew.-% ist die folgende:

| | |
|---|---|
| allgemeines Eiweiß | von 60 bis 63 |
| wirkliches Eiweiß | von 42 bis 46 |
| Kohlenhydrate | von 10 bis 13 |
| Lipide | von 4 bis 8 |
| Nukleinsäuren | von 3 bis 6 |
| Mineralstoffe | von 6 bis 8 |
| Vitamine | von 2,6 bis 3,1 |
| Wasser | Rest |

Einen Bestandteil der Eiweißkomponenten von MILAIF bilden alle essentiellen Aminosäuren (% zum allgemeinen Eiweiß): Lysin - 9,2; Isoleuzin - 3,4; Leuzin - 4,5; Phenylalanin + Thyrosin - 4,5; Cystin + Methionin - 2,3; Threonin - 3,7; Tryptophan - 0,8; Valin - 3,6, deren Anteil 35 bis 45 % von der Summe der Aminosäuren beträgt. Im Hinblick auf diesen Kennwert kommt MILAIF an die Muskeleiweiße heran, welche genauso 45 % essentieller Aminosäuren enthalten. Wie bekannt ist, spielen die Eiweißstoffe und die Derivate derselben eine große Rolle in den Stoffwechselprozessen der Verdauung, Atmung, Ausscheidung, Bewegung. Und sogenannte "essentielle" Aminosäuren müssen in den menschlichen Organismus in fertiger Form zugeführt werden, weil diese im Organismus nicht synthetisiert werden.

Der Kohlenhydratanteil von MILAIF, der 10-13 % bezogen auf die Trockensubstanz beträgt, besteht aus 1-3-β-D-Glykanen, die eine immunomodulierende Wirksamkeit zeigen (Kaschkin M.A., N.G. Elinow, "Immunomodulierende Wirksamkeit von Polysacchariden aus Pilzen", Zeitschrift "Mykologie und Phytopathologie", Band 19, Ausgabe 4, 1985, Seiten 1-3).

Die monomere Zusammensetzung dieser Glykane besteht aus Glucose, Galaktose, Mannose und Fruktose in einem Verhältnis von jeweils 1,0 : 1,38 :1,79 : 0,5.

In der Lipidfraktion von MILAIF; welche 4-8 % bezogen auf die Trockensubstanz, beträgt, sind physiologisch wirksame Stoffe verschiedenen Typs der Verbindungen: Phospholipide, Sterine, Glyzeride, Fettsäuren enthalten. Die Angaben über deren mengenmäßigen Gehalt sind in der Tabelle 1 dargestellt.

Die im MILAIF enthaltenen, physioligisch wirksamen Phospholipide (Phosphatidylserin, Phosphatidylethanolamin und Phosphatidyl-cholin) beteiligen sich an den verschiedenen Stoffwechselprozessen und dem Energieaustausch.

Phospholipide sind die hauptsächlichen Elemente in der Struktur der Zellwand und der Zellorganellen. Ihre funktionelle Bedeutung beruht auf der Regulierung der Durchlässigkeit von Zellwänden. Sie unterstützen die Arbeit des Zellmechanismus: Ionenaustausch, Atmung, biologische Oxydation, beeinflussen die Aktivität von Enzymen in Mitochondrien. Wegen des Mangels an Phospholipiden erfolgt die Störung des Fettstoffwechsels, was zur akuter Leberdegeneration führen kann.

In der Fettsäurenfraktion von MILAIF sind physiologisch wirksame Fettsäuren enthalten, die zu den essentiellen gehören. Über 50 % von der Summe der Fettsäuren entfällt auf den Anteil der Linolsäure, die eine Vorstufe der Prostaglandin-Regulatoren der Hormontätigkeit von Tieren und Menschen darstellt.

Dank ihrer bekannten pharmakologischen Eigenschaften regenerieren Phospholipide und ungesättigte Fettsäuren die beschädigten Mitochondrien, aktivieren gestörte Fermentsysteme, verstärken die Detoxikationsfunktion der Leber, analog zu dem bekannten Präparat ESSENTIALE (M. D. Maschkowsky, Arzneimittel, Verlag "Medizina", Moskau, 1984, Band 2, Seite 46).

Eine hohe physiologische Wirksamkeit besitzt 22,23-Dihydroergosterin, das ein Vitamin der Gruppe D (D₄) ist. Es zeigt die Aktivität, die dem Cholecalciferol (Vitamin D₃) ähnlich ist und trägt zu dem richtigen Phosphorcalciumstoffwechsel, insbesondere bei den wachsenden Organismen, und zur rechtzeitigen Ablagerung der Stoffe in den wachsenden Knochen bei (M. D. Maschkowsky, Arzneimittel, Verlag "Medizina", Moskau, 1984, Seite 34).

**Tabelle 1**

| Gehalt an physiologisch aktiven Komponenten in der Lipidfraktion von MILAIF (mg/100g Präparat) | | |
|---|---|---|
| Physiologisch aktive Stoffe | Mengenmäßiger Gehalt | |
| | von | bis |
| Phosphatidylserin | 0,12 | 0,28 |
| Phosphatidylethanolamin | 0,23 | 0,53 |
| Phosphatidylcholin | 0,79 | 0,85 |
| 22,23-Dihydroergosterin | 4,0 | 5,0 |
| Monoglyzeride | 46 | 92 |
| Triglyzeride | 1136 | 2272 |
| Oleinsäure C_{18:1} | 0,8 | 1,6 |
| Linolsäure C_{18:2} | 2,8 | 5,6 |
| Linolsäure C_{18:3} | 0,03 | 0,09 |
| Ubichinon Q 9 | 0,034 | 0,075 |
| Ubichinon Q 10 | 0,054 | 0,125 |
| Ubichinon Q₉ (Ubinon) ist ein Metabolit vom Organismus der Tiere und Ubichinon Q₁₀ - ein Metabolit des Menschen. | | |

Die vergleichenden Untersuchungen von Ubinon und Ubichinon Q₁₀ mit dem Vitamin E (dem aktiven antioxydativen Mittel) haben gezeigt, daß die antioxydante und anticytolytische Aktivität von Ubinon und Ubichinon Q₁₀ um das 5-fache höher war als beim Vitamin E. Ubinon und Ubichinon Q₁₀ wirken um das 5-fache aktiver im Vergleich zu dem Vitamin E und um das 1,5-fache aktiver im Vergleich zu der Liponsäure, normalisieren die Detoxikationsfunktion und die exkretorische Funktion der Leber bei der Beschädigung derselben mit Tetrachlorkohlenstoff.

Ubinon und Ubichinon Q₁₀ sind universelle unersetzliche Komponenten in der Funktion der biologischen Membranen und beim Metabolismus der Zellen im ganzen, darunter auch der Hepatozyten im Organismus von Tieren und Menschen. Ihre hohe Hepathoprotektoraktivität ist eine Folge vom Inhibieren der molekularen pathogenetischen Membranen-Mechanismen, die mit der Verstärkung der Peroxidoxydation von Lipiden zusammenhängen.

Aufgrund der bekannten experimentellen Angaben wurde festgestellt, daß Ubinon und Ubichinon Q#10 eine antioxydante Aktivität im Lebergewebe bei der toxischen Beschädigung desselben aufweisen. Ubinon und Ubichinon Q#10 verhüten Zytolyse und Nekrose von Hepatozyten und normalisieren die exkretorischen und detoxizierenden Leberfunktionen, die beim toxischen Hepatitis geändert sind (L. W. Winogradow, E. A. Obolnikowa u.a. "Ubichinone - perspektive Hepatoprotektoren mit einem metabolischen Wirkungstyp". Der Erste Russische Nationale Kongreß "Mensch und Arznei", April 1992, Moskau, Thesis 408).

Infolge der Analyse der Zusammensetzung der Lipidkomponente vom Präparat MILAIF kann man auf seine hohe physiologische Aktivität im Organismus schließen. Die mineralische Zusammensetzung von MILAIF ist in der Tabelle 2 angeführt.

**Tabelle 2**

| Mineralische Zusammensetzung von MILAIF | |
|---|---|
| Element, Konzentration | Lufttrockenes Präparat |
| Feuchtigkeit, % | 5,95 |
| Asche, % | 10,87 |
| Natrium, mg/g | 1,74 |
| Calcium, mg/g | 17,37 |
| Magnesium, mg/g | 2,92 |
| Phosphor, mg/g | 15,0 |
| Eisen, mkg/ | 64,0 |
| Zink, mkg/g | 18,0 |
| Kupfer, mkg/g | 10,0 |
| Mangan, mkg/g | 39,0 |
| Strontium, mkg/g | 4,76 |
| Cobalt, mkg/g | 2,0 |
| Nickel, mkg/g | 4,0 |
| Chrom, mkg/g | 3,0 |
| Lithium, mkg/g | unter 0,004 |
| Rubidium, mkg/g | 3,16 |
| Molybdän, mkg/g | 0,1 |
| Aluminium, mkg/g | 7,1 |
| Blei, mkg/g | 0,2 |
| Cadmium, mkg/g | 0,2 |
| Arsen, mkg/g | 0,1 |
| Quecksilber, mkg/g | unter 0,01 |

Aus den in Tabelle 2 angeführten Angaben folgt, daß in MILAIF eine beträchtliche Menge von physiologisch wirksamen Elementen enthalten ist.

Calcium beteiligt sich im Organismus an der Bildung des Knochengewebes, an der Aktivierung der Fermente der Blutgerinnung, an der Herabsetzung der Erregbarkeit von einzelnen Bereichen des Nervensystems, an der Abschwächung der Wirkung von Toxinen, an der Erhöhung der Beständigkeit gegen Infektion, an der Erregung der bioelektrischen Potentiale auf der Oberfläche der Zellmembranen.

Kalium stellt die Normalisierung des Innenzelldruckes sicher und ist ein Aktivator vieler Fermente.

Phosphor und seine Verbindungen dienen als Bestandteil des Knochengewebes und der Zähne, beteiligen sich an den Energieprozessen.

Kupfer nimmt an den Eisenumwandlungen in die Formen teil, welche für die Synthese vom Hämoglobin zugänglich sind.

Mangan beeinflußt die Synthese von Glykogen, nimmt an der Normalisierung des Kohlenhydratstoffwechsels teil.

Eisen bildet einen Bestandteil vom Hämoglobin, der Eisenmangel führt zu einer schweren Krankheit - Anämie.

Ein weites Spektrum der chemischen Elemente, die einen Bestandteil von MILAIF bilden, und moderne Vorstellungen über deren Rolle im Organismus des Menschen weisen somit die physiologische Wirksamkeit dieses Präparates nach.

Wasserlösliche Vitamine, die in MILAIF enthalten sind, sind in der Tabelle 3 dargestellt.

Im Präparat MILAIF sind Vitamine, hauptsächlich die der Gruppe B, enthalten, deren Menge summarisch 2,6 bis 3,1 % beträgt.

**Tabelle 3**

| Vitamingehalt im Präparat MILAIF | | |
|---|---|---|
| Vitamine, mg % | Menge | |
| | von | bis |
| B₁ (Thiamin) | 11 | 13 |
| B₂ (Riboflavin) | 56 | 64 |
| B₃ (Pantothensäure) | 38 | 49 |
| B₄ (Cholin) | 2300 | 2800 |
| B₆ (Pyridoxin) | 9 | 10 |
| Folsäure | 1 | 2 |
| PP (Nikotinsäure) | 206 | 231 |
| Biotin | 2 | 3 |
| B₁₂ (Cyanocobalamin) | 8 | 9 |

Die physiologische Wirkung der Vitamine der Gruppe B ist zur Zeit gut studiert. Es ist bekannt, daß sie eine wichtige Rolle im Eiweiß-, Fett- und Kohlenhydratstoffwechsel spielen, die Resistenz des Organismus gegen Infektionskrankheiten dank der immunostimulierenden Wirkung erhöhen, an der Synthese der Prostaglandinen, Fermente und Cofermente teilnehmen, eine effektive vorbeugende Wirkung besitzen. In MILAIF haben diese Vitamine einen natürlichen Ursprung und sind in Form eines Naturkomplexes anwesend, was ein beträchtlicher Vorteil im Vergleich zu den synthetischen Analoga derselben und den künstlich geschaffenen Kompositionen (Methiovit, Vitamin B₁) ist.

### Medizinisch-biologische Untersuchungen von MILAIF

Die Aufgabe der medizinisch-biologischen Untersuchungen von MILAIF bestand in der Erstellung von Angaben, die die Sicherheit seiner Verwendung bei heilenden und vorbeugenden Zwecken garantieren.

Nach den Untersuchungen, die an drei Tierarten (Ratten, Hühner, Schweine) durchgeführt wurden, wurde gezeigt, daß beim peroralen Eintritt in den Organismus MILAIF:
- keine negative Einwirkung unter den Bedingungen des akuten (30 Tage), subchronischen (60 Tage) und chronischen (1 Jahr) Experimentes bei seinem 10-, 25-, 50- und 100%igen Ersatz des Proteins vom Vivariumsfutterstoff besitzt;
   keine carcinogene Wirkung aufweist und keinen modifzierenden Einfluß auf die Entwicklung von spontanen und induzierten Geschwülsten ausübt;
   keine dominanten Letalmutationen in den Geschlechtszellen der Tiere hervorruft;
   keine teratogenetische Wirkung und Embriotrophiewirkung in fünf Generationen von Tieren zeigt;
   keine allergische Wirkung zeigt;
   keinen Einfluß auf das Gewicht von Herz, Leber, Nieren, Milz ausübt;
   den morphologischen Blutindex von Tieren nicht beeinflußt, die innerhalb der physiologischen Norm bleiben.

Im Prozeß der Untersuchung von MILAIF wurde die Erhöhung der Aktivität der Suczinatdehydrogenase und die Vergrößerung des Glykogengehaltes in der Leber von Ratten festgestellt, in deren Diät der Bedarf an Protein vollkommen durch MILAIF befriedigt wurde. Es wurde auch die Herabsetzung der Fettmenge in der Leber bei den Ratten festgestellt, die Protein nur mit MILAIF erhalten haben.

Ähnliche Ergebnisse wurden mit Schweinen erhalten, deren Fütterung nach den festgelegten physiologischen Normen erfolgte.

Untersucht wurden drei Tiergruppen:

Die erste Gruppe erhielt den üblichen Futterstoff und als Eiweißkomponente (30%) Knochenmehl.

Die zweite Gruppe erhielt ebenso wie die erste den gewöhnlichen Futterstoff und als Eiweißkomponente (30%) den MILAIF-Eiweißstoff.

In der Tabelle 4 sind die Ergebnisse dieser Untersuchungen angeführt.

**Tabelle 4**

| Kennwerte des Lipidstoffwechsels im Blutserum von Schweinen, die MILAIF erhalten haben | | |
|---|---|---|
| Kennwert, mg/l | 1. Gruppe (Knochenmehl) | 2. Gruppe (MILAIF) |
| Cholesterin | 1525 ± 53 | 1302 ± 109 |
| Phospholipide | 1390 ± 95,2 | 1305 ± 70,1 |
| Triglyzeride | 1238 ± 84 | 940 ± 112 |
| Allgemeine Lipide | 4440 ± 134 | 3770 ± 135 |
| β-Lipoproteide | 1200 ± 28 | 925 ± 70 |

Die erhaltenen Angaben zeugen davon, daß MILAIF einen günstigen Einfluß auf den Lipidstoffwechsel ausübt, indem er eine beträchtliche Verminderung des Gehaltes an Cholesterin und Triglyzeriden hervorruft.

### Klinische Untersuchung der therapeutischen Wirkung von MILAIF bei der alimentären Fettsucht (Störung vom Lipidstoffwechsel)

Es ist bekannt, daß die alimentäre Fettsucht unentbehrlich zur Entwicklung der Pathologie führt, die verschiedene Systeme des Organismus verletzt.

Im Zusammenhang damit erhielten die Kranken von drei gebildeten Gruppen MILAIF:
1. Kranke, die an Fettsucht in Kombination mit den Erkrankungen des metabolitischen Profils (Hypercholesterinämie, arterielle Hypertension, Zuckerkrankheit mit Verletzung der Glucosetoleranz) leiden - 45 Personen.
2. Kranke, die an Fettsucht in Kombination mit der Pathologie des Gallenabscheidungssystems (chronische Cholecystitis, Cholelithiasis, Dyskinäsie der ableitenden Gallenwege) leiden - 18 Personen.
3. Kranke, die an Fettsucht in Kombination mit der Pathologie des Magen-Darm-Kanals (Ulkuskrankheit, erosive Gastritis und Bulbitis, Duodenits) leiden - 12 Personen.

### 1. Untersuchung des Einflusses von MILAIF auf die Dynamik der klinischbiochemischen Indexzahlen bei den Kranken, die an Fettsucht in Kombination mit den Erkrankungen des metabolitischen Profils leiden

Überwacht wurden 45 Kranke, bei denen sich die Fettsucht mit Hypercholesterinämie vereinigte (1. Gruppe - Männer 20 Personen, 2. Gruppe - Frauen 25 Personen).

Die Untersuchung der herkömmlichen Indexzahlen des Lipidstoffwechsels, welche einschließen: Gehalt im Blutserum an allgemeinem Cholesterin (OChS), an Lipoproteiden mit einer hohen Dichte (LPWP), Triglyzeriden (TG), an Koeffizient der Atherogenität (KA), erfaßte bei den Kranken gesetzmäßige Verschiebungen dieser Kennwerte (Tabelle 5). Ein besonderes Interesse stellt die Analyse der Kennwerte dar, die das Risiko der Entwicklung der ischämischen Herzkrankheit (IBS) kennzeichnen, weil in der zu untersuchenden Gruppe die überwältigende Anzahl von Patienten (33 Personen) jünger als 40 Jahre war.

Der Wert der Indexzahl LPWP/OChS sowohl in der ganzen Gruppe als auch in den einzelnen Gruppen von Männern und Frauen wies darauf hin, daß die Patienten sich im Bereich eines hohen Risikos der Entwicklung dieser Pathologie befanden: 17,6-18,7 % bei der Norm von 20-25 %. Der Coeffizient der Atherogenität überschritt signifikant die normalen Werte (4,5 ± 0,4 im Vergleich zu der Norm von 3,0).

Bei 50 % der zu überwachenden Kranken sind zwei Risikofaktoren für die Entwicklung der ischämischen Herzkrankheit (Hypercholesterinämie und Hypertensie) festgestellt. Nach der durchgeführten Kur wurde eine ausgesprochene Verminderung der Werte von Lipidindexzahlen sowohl für die ganze Gruppe als auch für die beiden zu untersuchenden Gruppen festgestellt.

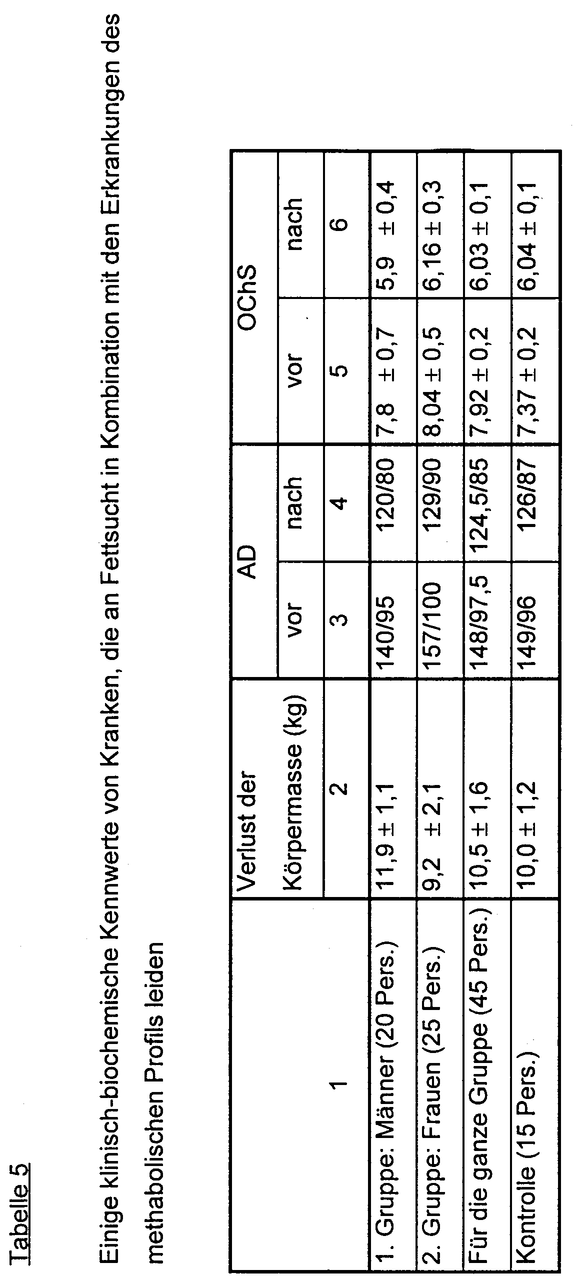

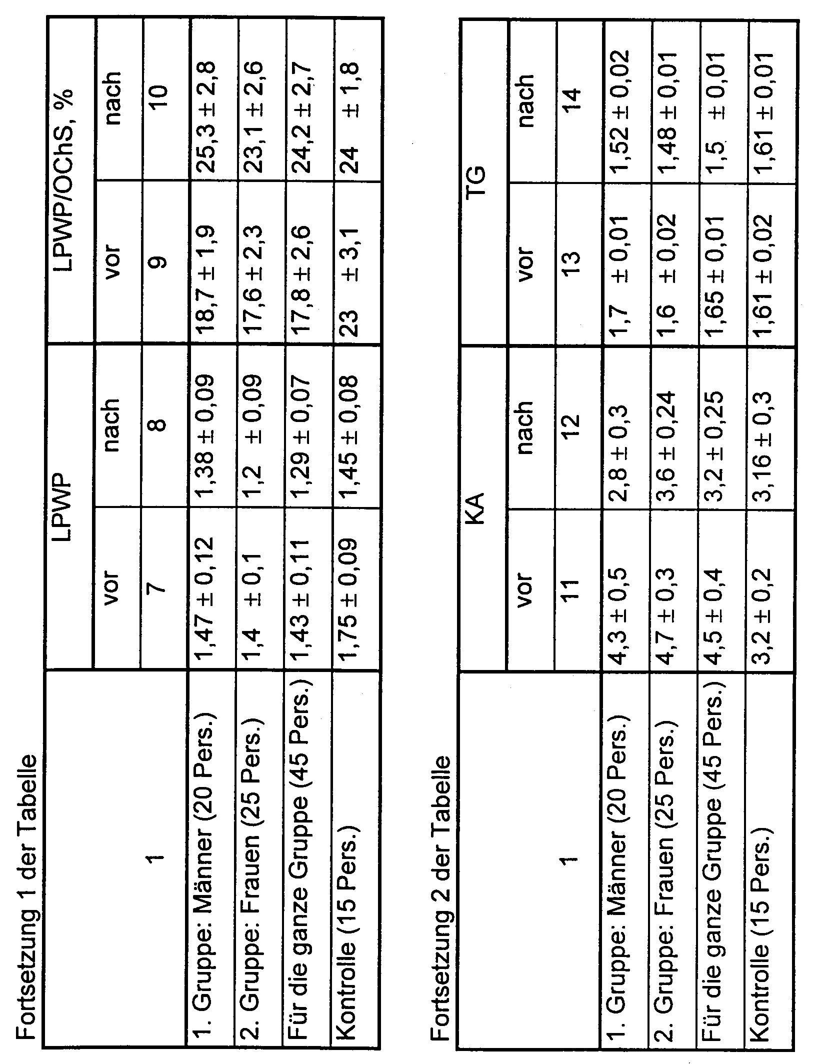

### Anmerkung:

AD = arterieller Druck
OChS = allgemeines Cholesterin
LPWP = Lipiproteide mit einer hohen Dichte
LPWP/OChS, % = Verhältnis zwischen LPWP und dem allgemeinen Cholesterin
KA = Koeffizient der Atherogenität
TG = Triglyzeride

Besonders ausgeprägte Änderungen wurden bei Männern festgestellt: statistisch bedeutungsmäßige Herabsetzung des Cholesterinspiegels, des Koeffizienten der Atherogenität, der Triglyzeride und Erhöhung des Wertes von LPWP/OChS.

Der Vergleich der erhaltenen Ergebnisse mit den Kontrollangaben zeigt einen mehr ausgeprägten positiven Effekt bei den Patienten, denen MILAIF verabreicht wurde. Die Einwirkung der herkömmlichen Therapie war weniger ausgedrückt.

Bei 15 Kranken wurden während der ersten Untersuchung die erhöhten Indexe des Zuckergehaltes im Blut festgestellt. Bei der sorgfältigen Untersuchung (Glykämiekurve mit der Belastung von 75 g der Glucose, Glykämische Indexe) wurde bei 5 Kranken Zuckerdiabetes vom zweiten Typ festgestellt, bei 10 Patienten - Störung der Glucosetoleranz. Es wurde eine statistisch signifikante Depression dieses Indexes in der Gruppe von Kranken, denen MILAIF verabreicht wurde, von 7,54 ± 0,9 mmol/l bis 5,3 ± 0,48 mmol/l im Vergleich zur Kontrolle 6,76 ± 0,7 mmol/l bis 6,32 ± 0,6 mmol/l gefunden.

Die Verträglichkeit des Präparates war bei allen Kranken gut. Bei 75 % von den zu überwachenden Personen wurde eine beträchtliche Verminderung des Hungergefühls und der Abfall des Appetits zu vermerken. Der Prozeß der Abnahme der Körpermasse verlief viel behaglicher. Der Verlust der Körpermasse bei Männern betrug 11,9 ± 1 kg und bei Frauen - 9,2 ± 2,1 kg.

### 2. Einfluß von MILAIF auf die Dynamik der klinisch-biologischen Kennwerte bei Kranken in Kombination mit der Pathologie des Hepatobilisystems

Beobachtet wurden 18 Kranken mit verschiedener Pathologie des Gallenabsonderungssystems. Bei 50 % der Kranken wurden funktionelle Diskinesiestörungen - hypomotorische Diskinäsie von Gallenabscheidungswegen - festgestellt. 50 % der Kranken hatten eine ausgeprägtere organische Pathologie - bei ihnen wurde das Gallensteinleiden diagnostiziert, was durch Röntgenuntersuchung bestätigt wurde. Alle Kranken erwähnten bei der Einlieferung ein Lastgefühl im Bereich des rechten Hypochondriums, anfallartige Schmerzen bei fehlerhafter Diät und nach schwerer physischer Belastung. Bei diesen Kranken wurden genauso verschiedene Verdauungsstörungen festgestellt: bitterer Geschmack in der Mundhöhle, Übelkeit nach dem Genuß von fetten Speisen. Die Kranken haben einen traditionellen Kurs der Diättherapie erhalten, der auf die Verminderung der Körpermasse unter Anwendung von galleabführenden Mitteln gerichtet war.

MILAIF wurde in dem durchgeführten Therapiekurs im Laufe von 21 Tagen miteingenommen. Die Verträglichkeit des Präparates war bei sämtlichen Patienten (ohne Ausnahme) gut. Bei 75 % der Kranken wurde eine Verminderung des Appetits festgestellt.

Die Dynamik der biochemischen Kennwerte ist in der Tabelle 6 zusammengefaßt.

**Tabelle 6**

| Dynamik der biochemischen Kennwerte bei den Kranken, die an Fettsucht in Kombination mit der Pathologie des Hepatobilisystems leiden | | | | | | | | |
|---|---|---|---|---|---|---|---|---|
| | Gallenfarbstoff | | OChS | | LPWP | | TG | |
| | vor | nach | vor | nach | vor | nach | vor | nach |
| 1 | 2 | 3 | 4 | 5 | 6 | 7 | 8 | 9 |
| Überwachte Kranke | 27,8 ± 3,1 | 20 ± 4 | 8,5 ± 0,7 | 5,9 ± 0,4 | 1,5 ± 0,1 | 1,2 ± 0,2 | 1,7 ± 0,09 | 1,5 ± 0,2 |
| Kontrolle | 24,6 ± 4,2 | 20,2 ± 3,8 | 7,4 ± 0,9 | 6,4 ± 1,0 | 1,24 ± 0,1 | 1,26 ± 0,2 | 1,54 ± 0,1 | 1,54 ± 0,3 |

Wie aus den eingereichten Angaben ersichtlich ist, sind bei den Kranken der gegebenen Gruppe der Pathologie die Abweichungen festzustellen, die für die ganze Gruppe der Korpulenten im großen und ganzen spezifisch sind. Die Erhöhung der Werte des Serumgallenfarbstoffes, die vom Gallenstauungssyndrom bedingt ist, hatte eine signifikanteTendenz zur Abnahme in der Gruppe der Kranken, denen das geprüfte Präparat verabreicht wurde. Es wurde genauso die Verminderung des Appetits, die Abnahme des Hungergefühls und die ausgeprägte Depression von Lipidkennwerten (OChS, LPWP, TG) beobachtet, was es erlaubt, die Verschreibung des Präparates der gegebenen Kategorie der Kranken für überaus zweckmäßig zu halten.

Auf solche Weise besitzt MILAIF die Eigenschaft der ausgeprägten Normalisierung der Indexzahlen von Cholesterin und Triglyzeriden im Blutserum bei den Kranken mit den kardiovaskulären Erkrankungen, die auf dem Hintergrund der Störungen des Lipidstoffwechsels ablaufen, und vermindert dadurch das Risiko der Entwicklung der ischämischen Herzkrankheit.

### Untersuchung der adaptogenetischen Wirkung von MILAIF

Die Untersuchungen wurden bei 15 onkologischen Kranken im Alter von 31 bis 65 Jahren mit unterschiedlicher Lokalisierung der Geschwülste (Gebärmutterhalskrebs der II. und III. Stufe, Nierenkrebs der III. Stufe, Lymphogranulomatose) durchgeführt. Als Kontrolle dienten 15 Personen im gleichen Alter und mit den gleichen Lokalisierungen von Geschwülsten. Alle Kranken wurden mit Einzeldosen von 2 bis 4 g. und mit einer Gesamtdosis von 40 bis 60 g. behandelt. Bei allen Kranken, denen MILAIF verabreicht wurde, war eine Verminderung der Leukopenie im Vergleich zu der Kontrollgruppe zu vermerken (um 1600 ± 370, P < 0,05). Sogar wenn der Ausgangsspiegel von Leukozyten niedrig war (1,7 x 10⁸ - 1,9 x 10⁸), gelang es, im Laufe einiger Tage diesen bis 3,0 x 10⁸ - 4,0 x 10⁸ und manchmal sogar bis 5,5 x 10⁸ zu erhöhen. Es gelang, eine Erhöhung der Leukozytenzahl bei der Ausnutzung auch der anderen Biostimulatoren zu erzielen, aber die Wirkung von MILAIF zeichnete eine große Stabilität des Effektes aus.

Die Erhöhung der Leukozytenzahl war stabil und wurde auf dem Hintergrund der fortdauernden Bestrahlung oft beobachtet, was äußerst selten vorkommt, besonders ohne Bluttransfusion. Es gelang, bei allen Kranken einen vollen Kurs der Bestrahlung ohne Unterbrechung, die von der Leukopenie hervorgerufen sein könnte, und ohne Bluttransfusion, durchzuführen.

Der Prozentgehalt an Monozyten war hoch, aber auch etwas niedriger als im Kontrollfall: bei 10 aus 15 Personen überschritten die Monozyten die Norm nur um 3 bis 5 %, während bei den übrigen auch im Kontrollfall eine bedeutende monozytäre Leukozytose (18 - 26 %) zu vermerken war.

Bei allen Kranken war die Verminderung der Schwäche zu beobachten, verbesserten sich die Eßlust, der Schlaf und die Stimmung.

Die durchgeführten Untersuchungen zeugen also davon, daß MILAIF ein Adaptationsmittel ist und den Organismus vor der Wirkung eines solchen schädigenden Faktors schützt wie wiederholter Bestrahlung.

Der Einfluß von MILAIF ist ebenso bei den Kranken mit der spontanen Leukopenie (5 Personen) und der Leukopenie als Folge der Strahlentherapie (11 Personen) untersucht. Die Indexzahlen wurden bei den Kranken untersucht, denen MILAIF im Laufe von 15, 30 und 60 Tagen verabreicht wurde (1. Etappe), und während einer wiederholten Behandlung nach 12 Monaten (2. Etappe). Die erhaltenen Ergebnisse sind in der Tabelle 7 zusammengefaßt.

**Tabelle 7**

| Einfluß von MILAIF auf die Leukozytenmenge im Prozeß einer dauernden Therapie von Kranken, die an Leukopenie leiden | | | | |
|---|---|---|---|---|
| Kranke | Leukozytenmenge vor Therapie | Therapiedauer, Tage | | |
| | | 15 | 30 | 60 |
| | | Leukozytenmenge nach der Therapie | | |
| 1 | 2 | 3 | 4 | 5 |
| 1. Etappe Gesamtzahl 16 Pers. | 3195 ± 166 | 3936 ± 167 | 4807 ± 183 | 4750 ± 164 |
| Leukopenie als Folge der R-Therapie 11 Pers. | 3405 ± 168 | 4010 ± 172 | 4807 ± 173 | 4800 ± 165 |
| Spontane Leukopenie 5 Pers. | 2700 ± 151 | 3733 ± 154 | 3733 ± 196 | 4500 ± 161 |
| 2. Etappe Therapie nach 12 Mon. Gesamtzahl 16 Pers. | 3310 ± 158 | 4060 ± 161 | 4803 ± 171 | 4750 ± 158 |
| Leukopenie als Folge der R-Therapie 11 Pers. | 3350 ± 157 | 4000 ± 160 | 4800 ± 171 | 4800 ± 157 |
| Spontane Leukopenie 5 Pers. | 2800 ± 160 | 4310 ± 165 | 4807 ± 172 | 4500 ± 160 |

Aus den Angaben der Tabelle 7 läßt sich eine deutliche Dynamik der Vergrößerung der Leukozytenzahl bei den Kranken, die an Leukopenie verschiedener Ätiologie leiden, zurückverfolgen.

Somit findet die Adaptogenwirkung von MILAIF ihren Niederschlag in der Erhöhung der nicht spezifischen Widerstandsfähigkeit des Organismus bei den Kranken, die an Leukopenie verschiedener Ätiologien leiden.

### Untersuchung der Einwirkung von MILAIF auf immune Prozesse

Es wurden Kinder im Alter von 3 bis 5 Jahren untersucht, die in einem im Hinblick auf die Ökologie ungünstigen Gebiet wohnen bei denen das Syndrom der Überoxydation festgestellt wurde. Es wurden insgesamt 77 Personen untersucht, von diesen bildeten 56 Personen die Hauptgruppe (1), 21 die Kontrollgruppe (2).

Den Kindern der Hauptgruppe verabreichte man täglich MILAIF im Laufe von 20 bis 25 Tagen.

Die Kontrollgruppe wurde in der Krankenstation standardmäßig behandelt.

Die Kinder der beiden Gruppen wurden sorgfältig vom Kinderarzt, Otorhinolaryngologen, Allergologen, Neuropathologen, Immunologen, Mikrobiologen und anderen Fachleuten untersucht.

Aus 56 Kindern der Hauptgruppe erwiesen sich 39 als sensibilisiert in Bezug auf die Gewebe von Innenorganen, und aus diesen bei 21 Kindern wurde chronische Gastritis diagnostiziert, bei 18 chronische Colitis mit den Erscheinungen der Dysbakteriose, bei 32 chronische Cholecystitis und Cholecystoangiocholitis.

Neben den allgemeingültigen Untersuchungen (allgemeine Blutanalyse, Harnanalyse, Kotanalyse, EKG) wurden mit Immunochemieluminiszenzmethoden Untersuchungen des Blutes und des Speichels zur Bestimmung von Antikörpern gegen Organe und Bakterien- auf dem Analysator "Beta-2" durchgeführt, welche auf dem sehr schwachen Leuchten von Biomedien und Zellen in dem sichtbaren Bereich desSpektrums beruhen, das durch die Lipidgebilde bedingt ist.

Die Änderungen der Kennwerte der Autoimmunisation infolge der Verwendung von MILAIF sind in der Tabelle zusammengefaßt.

**Tabelle 8**

| Dynamik der Kennwerte der Autoimmunisation bei Einnahme von MILAIF | | | | | |
|---|---|---|---|---|---|
| Antigenart gegen Organ | Intensität der Bildung von Autoantikörpern in verabredeten Einheiten der Chemieluminiszenz | | | | |
| | 1. Gruppe | | 2. Gruppe | | Kontrollgruppe ohne Antigen |
| | vor | nach | vor | nach | |
| 1 | 2 | 3 | 4 | 5 | 6 |
| Lunge | 516,2 ± 34,1 | 476,0 ± 50,6 | 496, 8 ± 26,1 | 401,2 ± 38 | 403,7 ± 30,8 |
| Milz | 1270,5 ± 126,6 | 617,1 ± 99,0 | 1121 ± 39,7 | 991 ± 79,2 | 403,7 ± 30,8 |
| Niere | 715,5 ± 40,0 | 512,8 ± 52,8 | 701,8 ± 52,3 | 501,8 ± 67,6 | 403,7 ± 30,8 |
| Leber | 1112,8 ± 36,5 | 618,8 ± 75,3 | 1122,9 ± 40,1 | 884,1 ± 36,2 | 403,7 ± 30,8 |
| Dünndarm | 910,8 ± 78,4 | 572,8 ± 60,4 | 924,8 ± 76,8 | 612,1 ± 39,9 | 403,7 ± 30,8 |
| Magen | 818,3 ± 35,4 | 490,0 ± 28,8 | 886,4 ± 32,8 | 727,3 ± 31,4 | 403,7 ± 30,8 |
| Bauchspeicheldrüse | 701,4 ± 16,8 | 518 ± 50,5 | 694,1 ± 18,6 | 498,8 ± 39,7 | 403,7 ± 30,8 |
| Herzen | 516,8 ± 40,2 | 503,8 ± 60,4 | 598,7 ± 40,2 | 412,1 ± 26,3 | 403,7 ± 30,8 |
| Dickdarm | 854,6 ± 25,3 | 454,7 ± 36,8 | 832,1 ± 33,1 | 511,1 ± 36,4 | 403,7 ± 30,8 |

Aus den Angaben der Tabelle 8 ist ersichtlich, daß sich bei den Kindern sowohl der Hauptgruppe als auch der Kontrollgruppe im Blut eine beträchtliche Menge der Antiorganantikörper ansammelt und sich eine ausgeprägte Autoimmunisation zu den Geweben von Milz, Leber, Dick- und Dünndarm sowie Magen herausstellt. Der Vergleich der Therapieergebnisse der Hauptgruppe und der Kontrollgruppe hat gezeigt, daß die Verminderung der Intensität der Autoimmunisation bei den Kindern der Kontrollgruppe in Bezug auf die Verdauungsorgane weniger ausgeprägt war und die Intensität in Bezug auf die Gewebe von Milz, Lunge und Herzen nicht signifikant vermindert wurde.

Die Verschreibung von MILAIF den Kindern der Hauptgruppe im Laufe von 20-25 Tagen trug zur signifikanten Verminderung der Menge der im Blut und den Geweben von Milz, Niere, Leber, Magen und Darm zirkulierenden autoimmunen Antikörper bei.

Es ist also zweckmäßig, MILAIF in den Fällen der Erkrankung der Verdauungsorgane zu verschreiben, die von dem autoimmunen Prozeß und der Schwächung der Schutzkräfte des Organismus begleitet werden.

Untersucht ist der Einfluß von MILAIF auf die Mikroflora bei den Kindern aus den gleichen Gruppen.

Bei 54,2% der Kinder wurde die Störung eines oder mehrerer Indexzahlen festgestellt, die die Hautflora kennzeichnen, bei 38,2 % kam eine erhöhte Menge der Mikroorganismen pro Einheit der Hautoberfläche vor, bei 36,8 % wurden Mannitzerfallende Bakterien festgestellt, und bei 44,5 % - hämolytische Bakterien, die bei gesundem Organismus des Kindes nicht vorhanden sind.

Die Störungen der Mikroflora des Darmes wurden durch eine wesentliche Reduzierung der Vermehrungsfähigkeit von Bifidusbakterien, durch die Sichtbarmachung des Colibakteriums mit einer geschwächten fermentativen Aktivität, der Colibazillen in einer laktosenegativen Form sowie durch die Änderung der normalen Verhältnisse zwischen den verschiedenen Vertretern der Darmflora als Folge der Erscheinung einer großen Menge der Coccoformen vom Typ Staphylococcus albus und Streptococcus gekennzeichnet.

Bei 6,4 % der Kinder sind in den Inokulationsfäkalien Pilze der Art CANDIDA im aktiven Zustand registriert.

Deshalb war es notwendig, die Entwicklung der Sensibilisierung oder der bakteriellen Infizierung der Kinder mit der pathogenen Autoflora sowie die Möglichkeiten der Behandlung derselben mit MILAIF zu studieren.

Sensibilisierung an bakterielle Allergene wurde nach der Methode der Immunochemieluminiszenz untersucht.

Es wurde die Ansammlung der bakteriellen Antikörper im Blut und im Speichel in Bezug auf die Antigene vom Streptococcus, Staphylococcus, Kolibakterium, Proteus, Enterococcus, Pyozyaneusbakterie bestimmt.

Die Untersuchung der bakteriellen Infizierung und der bakteriellen Sensibilisierung erlaubten es, die immunologischen Reaktionen in Bezug auf die bakteriellen Antigene zu erfassen.

Am häufigsten sowohl bei den Kindern der Hauptgruppe als auch der Kontrollgruppe war die Sensibilisierung gegen Colibakterien, Staphylococcus und Proteus zu vermerken.

Es hat sich herausgestellt, daß der Umbau der Mikrobenumgebung bei den Kindern in der Regel mit der Intensivierung der Produktion von Antikörpern in Bezug auf einige Bakterien gleichzeitig begleitet wurde.

In der Tabelle 9 sind die Angaben angeführt, die bei der Untersuchung der Immunreaktionen in Bezug auf die bakteriellen Antigene bei den Kindern der Hauptgruppe und der Kontrollgruppe erhalten sind.

**Tabelle 9**

| Dynamik des Spiegels der Sensibilisation an die bakteriellen Antigene | | | | | |
|---|---|---|---|---|---|
| Art des bakteriellen Antigens | Intensität der Chemieluminiszenz (verabredete Einheiten) | | | | |
| | Probe ohne Antigen | 1. Gruppe | | 2. Gruppe | |
| | | vor der Behandlung | nach der Behandlung | vor der Behandlung | nach der Behandlung |
| 1 | 2 | 3 | 4 | 5 | 6 |
| Staphylococcus hämolyticus | 165 ± 41 | 567 ± 75 | 263 ± 41 | 582 ± 76 | 389 ± 71 |
| Streptococcus hämolyticus | 205 ± 77 | 382 ± 72 | 211 ± 34 | 401 ± 78 | 218 ± 78 |
| Colibakterium | 204 ± 46 | 701 ± 80 | 405 ± 48 | 688 ± 81 | 512 ± 73 |
| Proteus | 189 ± 56 | 590 ± 58 | 519 ± 76 | 548 ± 49 | 521 ± 38 |
| Pyocyaneusbakterie | 178 ± 45 | 443 ± 48 | 297 ± 68 | 463 ± 44 | 366 ± 48 |
| Enterecoccus | 211 ± 64 | 372 ± 52 | 228 ± 39 | 370 ± 56 | 254 ± 67 |

Aus den Angaben der Tabelle folgt, daß die Einnahme von MILAIF bei den Kindern, die zu der Hauptgruppe zugerechnet wurden, zur ausgeprägten Verminderung der Konzentration der Antikörper gegen Staphylococcus hämolytikus, Streptococcus, Colibakterium, Enterecoccus und Pyocyaneusbakterie beigetragen hat.

Es ist also zweckmäßig, MILAIF den Kranken mit den Erscheinungen der Dysbakteriose zu verschreiben, die sich auf dem Hintergrund der Unterdrückung der Schutzkräfte des Organismus entwickelten.

Auf solche Weise trug die Verwendung von MILAIF bei den Kranken, die an chronischen Cholecystoangiocholiten, Gastriten, Gastroduodeniten, Koliten, oft mit Erscheinungen der Dysbakteriose, die sich auf dem Hintergrund des Syndroms der Peroxydation entwickelte, litten, zur Verminderung der Ausprägung von autoimmunen Reaktionen, hauptsächlich auf Antigene des Magen-Darm-Kanals, und Hyposensibilisierung gleichzeitig an einigen bakteriellen Allergenen bei.

### Praktische Anwendbarkeit

Der Stamm des Pilzes F. SAMBUCINUM WSB-917 wird für die industrielle Herstellung des Präparates MILAIF verwendet.

Das anmeldungsgemäße Präparat wird seine Anwendung im Gesundheitswesen finden und kann verwendet werden als:
- zusätzliches Therapiemittel bei Störungen des Stoffwechsels von Lipiden, Cholesterin und Glucose (Atherosklerose, ischämische Herzkrankheit, Leberkrankheiten, Fettsucht, Diabetes zweiten Typs), insbesondere in der Gerontologie;
- prophylaktisches Mittel für somatische Erkrankungen und Komplikationen, die sich auf dem Hintergrund der Strahlentherapie bei onkologisch Kranken entwickeln;
- Hilfsmittel für die Durchführung der Allgemeinstärkungsbehandlung und der Desintoxikationsbehandlung;
- prophylaktisches Mittel für somatische Erkrankungen bei Personen, die unter im Hinblick auf die Ökologie komplizierten Verhältnissen und in Zonen mit erhöhter Strahlung leben und arbeiten;
- Mittel für die Behandlung von Dysbakteriosen
- Mittel für die Korrektur der Spurenelemente.

Unter Berücksichtigung der autoimmunen Aktivität ist die Verwendung des Präparates auch bei der Transplantation von Organen und Geweben möglich.

### Beschreibung des Verfahrens zur Herstellung des Präparates MILAIF

Der Herstellungsprozeß des Präparates umfaßt die folgenden Stufen:
- Vorbereitung und Sterilisation von flüssigem Nährboden;
   Zubereitung des Inokulationsmyzels vom Pilz;
   Züchtung vom Myzel in Arbeitsfermentationsapparaten;
   Konzentration, Isolierung, Trocknung und Auswiegen des Präparates.

Als Kohlenstoffquelle für das Wachsen des Myzels können verwendet werden: Zuckerrüben- und Zuckerrohrmelassen, Rohzucker, Saccharose, Glucose, technologische Abfallprodukte der Alkohol- und Cognacproduktion (Alkohol-und Cognacschlempen), Milchserum, Säfte von Hackfrüchten und Grünmasse. Als Quellen von Stickstoff, Kalium und Phosphor verwendet man Ammoniumnitrat und saures Kaliumphosphat.

Die Sterilisation des Nährbodens wird mit Hilfe einer Anlage der kontinuierlichen Sterilisation bei einer Temperatur von 125 ± 2 °C durchgeführt, die Verweilzeit in der Sterilisationszone beträgt 3 Minuten. Das Inokulationsmyzel für den Arbeitsfermentationsapparat wird zuerst in den Prüfgläsern auf einem festen Nährboden, dann in den Kolben auf dem Schwinggerät auf einem flüssigen Nährboden und danach im Inokulator unter den folgenden technologischen Verhältnissen gezüchtet:

| | |
|---|---|
| Temperatur | 25 ± 2 °C |
| pH-Wert | 5,5 - 6,2 |
| freie Lüftung | 1 m³/m³/min. |

Das Inokulationsmaterial ist fertig, wenn die Konzentration der Trockensubstanzen des Myzels im Aufzuchtmedium 9-12 g/l erreicht. 5 bis 10 Vol.-% eines solchen Mediums dienen als Inokulationsmaterial, das unter Einhaltung der Regeln der Aseptik in den Arbeitsfermentationsapparat gebracht wird.

Die Züchtung des Myzels im Arbeitsapparat erfolgt unter den gleichen Verhältnissen wie auch im Inokulator, auf dem Nährboden mit der gleichen Zusammensetzung. Es ist möglich, auch einen diskontinuierlichen Prozeß durch Abnehmen von Produkt und Nachfüllen von Nährboden (semikontinuierlich) sowie einen kontinuierlichen Prozeß der Züchtung durchzuführen.

Die nachfolgende Stufe des technologischen Prozesses ist:
- Eindickung des Aufzuchtmediums mit Hilfe von Vakuumfilterapparaten. Ferner erfolgt die Entwässerung des Produktes mit Hilfe von Infrarot-Trockenapparaten unter den schonenden Verhältnissen (bis +50 °C), unter welchen die biologische Wirksamkeit von Komponenten erhalten bleibt.

Die Dauer der Trocknung beträgt 15 bis 20 Minuten.

Ferner wird das Präparat granuliert, tablettiert, ausgewogen und verpackt.

Das auf solche Weise erhaltene Präparat kann ohne Verlust der Aktivität 24 Monate bei einer Raumtemperatur von 17 bis 25 °C gelagert werden.

### Beispiele, die die anmeldungsgemäße Zusammensetzung des Präparates MILAIF veranschaulichen:

### Beispiel 1

Das Pilzmyzel wurde unter den Bedingungen der semikontinuierlichen Kultivierung in dem Fermentationsapparat mit einem Rauminhalt von 200 l gezüchtet, unter Ausnutzung des nachfolgenden Nährbodens in Gew.-%:

| | |
|---|---|
| Rübenmelasse | 4 (2 im Hinblick auf reduzierende Stoffe), |
| Ammoniumnitrat | 0,3, |
| Saures Kaliumphosphat | 0,2, |
| Wasser | bis 100 |
| pH-Wert | 5,5 - 5,8. |

Das Inokulationsmaterial wurde in einer Menge von 5 Vol.-% eingebracht, die Temperatur der Kultivierung betrug 24 ± 1 °C, die Dauer eines Zyklus der Kultivierung - 14 Stunden. Der ASB-Inhalt betrug 20 g in 1 l des Mediums. Die Produktivität des Prozesses ist 1,4 kg/m³/h.

Das auf solche Weise hergestellte Präparat hatte die nachfolgende Zusammensetzung in Masse-%:

| | |
|---|---|
| Allgemeines Eiweiß (N x 6,25) | 60 |
| Kohlenhydrate | 13 |
| Lipide | 8 |
| Nukleinsäuren | 3 |
| Mineralstoffe | 8 |
| Vitamine | 2,6 |
| Wasser | 5,4 |

### Beispiel 2

Das Myzel wurde unter den Bedingungen der semikontinuierlichen Kultivierung in den Fermentationsapparaten mit einem Rauminhalt von 30 l auf einem Nährboden gezüchtet, der die nachfolgende Zusammensetzung in Gew.-% aufweist:

| | |
|---|---|
| Rohzucker | 2 |
| Ammoniumnitrat | 0,3 |
| Saures Kaliumphosphat | 0,2 |
| Maisquellwasser | 0,05 |
| Wasser | bis 100 |
| pH-Wert | 5,7 - 6,0 |

Das Inokulationsmaterial wurde in einer Menge von 8 Vol.-% eingebracht, die Temperatur der Kultivierung - 25 ± 1 °C.

Die Dauer eines Zyklus betrug 13 Stunden. Ferner nahm man das Abgießen des Aufzuchtmediums im Volumen von 92 % vor, brachte einen neuen Nährboden ein und führte den Züchtungsprozeß weiter; nach 13 Stunden wiederholte man die Arbeitsoperation. Die Konzentration von ASB betrug 20,1 g in l.

| | |
|---|---|
| Prozeßproduktivität | 1,5 kg/m³/h |

Das auf solche Weise hergestellte Präparat wies die nachfolgende Zusammensetzung in Masse-% auf:

| | |
|---|---|
| Allgemeines Eiweiß (N x 6,25) | 63 |
| Kohlenhydrate | 10 |
| Lipide | 4 |
| Nukleinsäuren | 6 |
| Mineralstoffe | 6 |
| Vitamine | 3,1 |
| Wasser | 7,9 |

### Beispiel 3

Die Züchtung vom Myzel führte man ebenso wie im Beispiel 2 durch, ausgenommen, daß das Inokulationsmaterial in einer Menge von 10 Vol.-% eingebracht wurde. Die Temperatur der Züchtung betrug 26 ± 1 °C, pH-Wert des Mediums 6,0 - 6,2.

In diesem Fall betrugen die Dauer der Kultivierung 12,5 Stunden und die Speicherung von ASB - 20,2 g in l.

Die Produktivität des Prozesses betrug 1,6 kg/m³/Stunde.

Das Präparat wies die nachfolgende Zusammensetzung in Masse-% auf:

| | |
|---|---|
| Allgemeines Eiweiß (N x 6,25) | 61,5 |
| Kohlenhydrate | 11,5 |
| Lipide | 6 |
| Nukleinsäuren | 4,5 |
| Mineralstoffe | 7 |
| Vitamine | 2,8 |
| Wasser | 6,7 |

## Patentansprüche

1. Verwendung des Stammes vom Pilz FUSARIUM SAMBUCINUM FUCKEL VAR. OSSICOLUM, (BERK. ET CURT.) BULAI WSB-917 (Allunionskollektion von Industriemikroorganismen des Instituts WNIIgenetika, die Kollektionsnummer WKPM F-169) als Produzent physiologisch aktiver Stoffe mit prophylaktischer und therapeutischer Zweckbestimmung.

2. Präparat für Prophylaxe und Therapie von Erkrankungen, die mit der Störung des Stoffwechsels zusammenhängen, das eine Adaptogen- und Immunomodulationswirkung besitzt, **dadurch gekennzeichnet,** daß es infolge der Entwässerung des Aufzuchtmediums enthalten ist, das im Prozeß der Flüssigphasen-Tiefen-Kultivierung der Monokultur des Pilzes F. SAMBUCINUM gebildet wird, die nachfolgende Zusammensetzung in Gew.-% aufweist:
| | |
|---|---|
| Allgemeines Eiweiß (N x 6,25) | von 60 bis 63, |
| Kohlenhydrate | von 10 bis 13, |
| Lipide | von 4 bis 8, |
| Nukleinsäuren | von 3 bis 6, |
| Mineralstoffe | von 6 bis 8, |
| Vitamine | von 2,6 bis 3,1, |
| Wasser | Rest, |
die Eigenschaft der Normalisierung der Indexzahlen von Cholesterin und Triglyzeriden im Blutserum der Kranken mit kardiovaskulären Erkrankungen, die auf dem Hintergrund der Störungen vom Lipidstoffwechsel verlaufen, besitzt, ein Risiko der Entwicklung der ischämischen Herzkrankheit vermindert, die spezifische Widerstandsfähigkeit des Organismus erhöht und ein Adaptionsmittel zum Schutz gegen eine wiederholte Strahlung ist, zur Vergrößerung der Leukozytenzahl bei Leukopenien beiträgt, die Ausprägung der autoimmunen Prozesse vermindert, hauptsächlich bezüglich der Antigene des Magen-Darm-Kanals, und ein Hyposensibilisierungsmittel zu den bakteriellen Allergenen darstellt.

3. Präparat nach Anspruch 2, **dadurch gekennzeichnet,** daß es in Form von Tabletten, Granulen oder Pulver hergestellt wird.

4. Präparat nach Anspruch 2, **dadurch gekennzeichnet,** daß als Träger des Präparates verschiedene Nahrungsmittel auftreten können.

## Claims

1. Use of the strain of fungus FUSARIUM SAMBUCINUM FUCKEL VAR. OSSICOLUM, (BERK. ET CURT.) BULAI WSB-917 (Allunion's collection of industrial microorganisms of the Institute WNIIgenetika, collection number WKPM F-169) as producer of physiologically active materials with prophylactic and therapeutic purpose.

2. Preparation for prophylaxis and treatment of diseases associated with disturbance of metabolism which has an adaptogenic and immunomodulation effect, characterised in that it is present due to dehydration of the cultivation medium which is formed in the process of liquid phase deep cultivation of the monoculture of the fungus F. SAMBUCINUM and has the following composition in wt.%:
| | |
|---|---|
| General albumen (N x 6.25) | from 60 to 63, |
| Carbohydrates | from 10 to 13, |
| Lipids | from 4 to 8, |
| Nucleic acids | from 3 to 6, |
| Mineral materials | from 6 to 8, |
| Vitamins | from 2.6 to 3.1, |
| Water | remainder, |
has the property of normalisation of the index numbers of cholesterol and triglycerides in the blood serum of patients with cardiovascular diseases, which proceed against the background of disturbances of lipid metabolism, reduces the risk of developing ischaemic heart disease, increases the specific resistance of the organism and is an adaptation agent for protecting against repeated radiation, contributes to increasing the leucocyte number in leucopenia, reduces the intensity of autoimmune processes, mainly regarding the antigens of the gastro-intestinal tract, and is a hyposensitising agent for the bacterial allergens.

3. Preparation according to claim 2, characterised in that it is produced in the form of tablets, granules or powder.

4. Preparation according to claim 2, characterised in that different nutrients may act as substrates for the preparation.

## Revendications

1. Utilisation de la souche de champignons FUSARIUM SAMBUCINUM FUCKEL VAR. OSSICOLUM (BERK. ET CURT,) BULAI WSB-917, (collection Allunion de micro-organismes industriels de l'institut WNIIgenetika, N° de collection WKPMF-169) pour produire des substances physiologiquement actives avec des propriétés prophylactiques et thérapeutiques.

2. Préparation prophylactique et thérapeutique qui est active contre des maladies ayant leur origine dans des troubles métaboliques et qui a une activité adaptogène et immunomodulatrice, caractérisée en ce que, par suite d'une déshydratation du milieu de culture obtenu consécutivement à une monoculture du champignon F. SAMBUCINUM immergé en phase liquide, on obtient la composition suivante, en % en poids :
| | |
|---|---|
| Protéines totales (N x 6,25) | de 60 à 63 |
| Hydrates de carbone | de 10 à 13 |
| Lipides | de 4 à 8 |
| Acides nucléiques | de 3 à 6 |
| Matières minérales | de 6 à 8 |
| Vitamines | de 2,6 à 3,1 |
| Eau | complément à 100 |
qui a la propriété de ramener à un niveau normal le taux de cholestérol et des triglycérides dans le sérum du sang de malades atteints de maladies cardio-vasculaires dues à des perturbations du métabolisme des lipides, de diminuer les risques de développement de maladies cardiaques ischémiques, d'améliorer les défenses spécifiques de l'organisme, de constituer un milieu d'adaptation assurant une protection contre des irradiations répétées, d'augmenter le nombre de leucocytes en cas de leucopénie, de diminuer l'intensité de processus auto-immunitaires, en particulier contre les antigènes du canal gastro-intestinal et de constituer un moyen d'hyposensibilisation vis-à-vis des allergènes bactériens.

3. Préparation selon la revendication 2, caractérisée en ce qu'elle se présente sous la forme de comprimés, de granulés ou de poudre.

4. Préparation selon la revendication 2, caractérisée en ce que le support de la préparation peut être constitué par différents produits alimentaires.
